# EUROPEAN PATENT SPECIFICATION

(11) **EP 3 541 316 B1**
(45) Date of publication and mention of the grant of the patent: **04.05.2022**
(21) Application number: 17804360.0
(22) Date of filing: 31.10.2017
(51) Int. Cl.: A61B 90/50, A61F 2/24, A61F 2/95

(54) **STABILIZATION AND ADVANCEMENT SYSTEM FOR DIRECT AORTIC TRANSCATHETER AORTIC VALVE IMPLANTATION**
STABILISIERUNG UND VORSCHUBSYSTEM ZUR DIREKTEN AORTALEN TRANSKATHETER-AORTENKLAPPEN-IMPLANTATION
SYSTÈME DE STABILISATION ET D'AVANCEMENT POUR IMPLANTATION DE VALVULE AORTIQUE PAR VOIE TRANSCATHÉTER AORTIQUE

(30) Priority: 15.11.2016 US 201615351610
(43) Date of publication of application: 25.09.2019
(73) Proprietor: Medtronic Vascular Inc., Santa Rosa, CA 95403 (US)
(72) Inventor: JONES, Traci, Santa Rosa California 95403 (US); MATTISON, Lars, Santa Rosa California 95403 (US); ADAMEK-BOWERS, Jasper, Santa Rosa California 95403 (US); WILLIAM, Paul Jr., Santa Rosa California 95403 (US); IAIZZO, Paul, Santa Rosa California 95403 (US); DUFFY, Angela, Santa Rosa California 95403 (US); BATEMAN, Michael, Santa Rosa California 95403 (US); STARKSON, Nathan, Santa Rosa California 95403 (US); NEWALKAR, Aditya, Santa Rosa California 95403 (US)
(74) Representative: Zimmermann & Partner Patentanwälte mbB
(86) International application number: PCT/US2017/059375
(87) International publication number: WO 2018/093565

(56) References cited:
- WO-A1-2016/166828
- US-A1- 2006 253 109
- US-A1- 2009 023 985
- US-A1- 2012 065 470
- US-A1- 2012 253 120
- US-A1- 2014 188 130
- US-A1- 2014 243 963
- US-A1- 2015 272 733
- US-A1- 2016 262 883

## Description

### FIELD OF THE INVENTION

The present invention relates to systems for percutaneous transcatheter delivery and implantation of a stent or valve stent. More particularly, the present invention relates to a stabilization system for direct aortic transcatheter aortic valve implantation (TAVI).

### BACKGROUND OF THE INVENTION

Heart valves are sometimes damaged by disease or by aging, resulting in problems with the proper functioning of the valve. Surgical heart valve replacement is typically performed for patients suffering from valve dysfunctions. Traditional open surgery inflicts significant patient trauma and discomfort, requires extensive recuperation times, and may result in life-threatening complications. US 2014/243963 A1 discloses a stabilization and advancement system for implantation of an annuloplasty ring.

To address the above mentioned concerns, efforts have been made to perform cardiac valve replacements using minimally invasive techniques. In these methods, laparoscopic instruments are employed to make small openings through the patient's ribs to provide access to the heart. While considerable effort has been devoted to such techniques, widespread acceptance has been limited by the clinician's ability to access only certain regions of the heart using laparoscopic instruments.

Still other efforts have been focused upon percutaneous transcatheter (or transluminal) delivery and implantation of replacement cardiac valves to solve the problems presented by traditional open surgery and minimally invasive surgical methods. In such methods, a valve stent is compacted for delivery in a shaft and then advanced, for example through an opening in the femoral artery, and through the vasculature to the heart, where the valve stent is then deployed in the valve annulus (e.g., the mitral valve annulus). While femoral artery access is typical for most percutaneous transcatheter delivery, patients with contra-indicative anatomy, such as extreme tortuous and/or narrow vasculature, pervious surgeries, or moderate to severe calcification require a different access point. In such cases, one such alternate access method is direct aortic TAVI. However, clinicians face several challenges when utilizing direct aortic TAVI methods including a lack of vascular support for the delivery system, a lack of dedicated tools, utilizing a different delivery system than femoral access TAVI, a non-standard procedure room orientation, a need for additional personnel, and close proximity to fluoroscopy.

Accordingly, there is a need for an improved system for direct aortic TAVI that includes increased stability of the introducer, accurate valve positioning, increased clinician safety, reduced manpower requirements, and increased ease of use.

### BRIEF SUMMARY OF THE INVENTION

Embodiments hereof relate to a stabilization and advancement system for transcatheter aortic valve implantation. The stabilization and advancement system of the invention includes an introducer support, a cradle mechanism, an adjustment mechanism and a flexible position clamp as claimed in claim 1. Further embodiments of the invention are defined by the dependent claims.

The introducer support is coupled to an introducer of a delivery system and to a stable object in a procedure room. The cradle mechanism is coupled to a handle of the delivery system and to a stable object in the procedure room. The adjustment mechanism is coupled to the delivery system. When operated, the adjustment mechanism moves the delivery system distal of the handle in a distal or proximal direction.

### BRIEF DESCRIPTION OF DRAWINGS

FIG. 1 is an illustration of a stabilization and advancement system according to an embodiment hereof.
FIG. 2 is an illustration of an introducer support of the stabilization and advancement system of FIG. 1.
FIG. 3 is a perspective illustration of the introducer support of FIG. 2.
FIG. 4 is a close-up illustration of a proximal end of the introducer support of FIG. 2 with a lock ring thereof in a first configuration.
FIG. 5 is a close-up illustration of the proximal end of the introducer support of FIG. 2 with the lock ring in a second configuration.
FIG. 6 is an illustration of a flexible position clamp of the introducer support assembly of FIG. 1.
FIG. 7 is a perspective illustration of a cradle the stabilization and advancement system of FIG. 1 according to an embodiment hereof.
FIG. 8 is an exploded view of the cradle of FIG. 7.
FIG. 9 is a perspective illustration of an adjustment mechanism of the stabilization and advancement system of FIG. 1 in a first configuration, according to an embodiment hereto.
FIG. 10 is a perspective illustration of the adjustment mechanism of the FIG. 9 in a second configuration.
FIG. 11 is a perspective illustration of a base and tube of the adjustment mechanism of FIG. 9.
FIG. 12 is a perspective illustration of a hinge of the adjustment mechanism of FIG. 9.
FIG. 13 is a cutaway side illustration of the adjustment mechanism of FIG. 9.
FIG. 14 is an end illustration of a thumbwheel and a wheel of the adjustment mechanism of FIG. 9.
FIG. 15 is an overhead illustration of the stabilization and adjustment mechanism of FIG. 1 coupled to a stable object, and configured with a delivery system for a direct aortic TAVI procedure on a patient.
FIG. 16 is a perspective illustration of another embodiment of an adjustment mechanism with an adjustment portion of the adjustment mechanism in a first configuration.
FIG. 17 is a perspective illustration of the adjustment mechanism of the FIG. 16 with the adjustment portion in a second configuration.
FIG. 18 is a close-up illustration of a proximal end of the adjustment mechanism of FIG. 16.
FIG. 19 is a top-view illustration of the adjustment mechanism of FIG. 16.
FIG. 20 is a perspective illustration of another embodiment of an adjustment mechanism, wherein an adjustment portion of the adjustment mechanism is in a first configuration.
FIG. 21 is a perspective illustration of the adjustment mechanism of the FIG. 20 with the adjustment portion in a second configuration.
FIG. 22 is an illustration of a proximal end of the adjustment mechanism of FIG. 20 with the adjustment portion in the second configuration.

### DETAILED DESCRIPTION OF THE INVENTION

Specific embodiments of the present invention are now described with reference to the figures, wherein like reference numbers indicate identical or functionally similar elements. The terms "distal" and "proximal", when used in the following description to refer to a delivery system or stabilization and advancement system are with respect to a position or direction relative to the treating clinician. Thus, "distal" and "distally" refer to positions distant from, or in a direction away from the treating clinician, and the terms "proximal" and "proximally" refer to positions near, or in a direction toward the clinician.

The following detailed description is merely exemplary in nature and is not intended to limit the invention or the application and uses of the invention. Furthermore, there is no intention to be bound by any expressed or implied theory presented in the preceding technical field, background, brief summary, or the following detailed description.

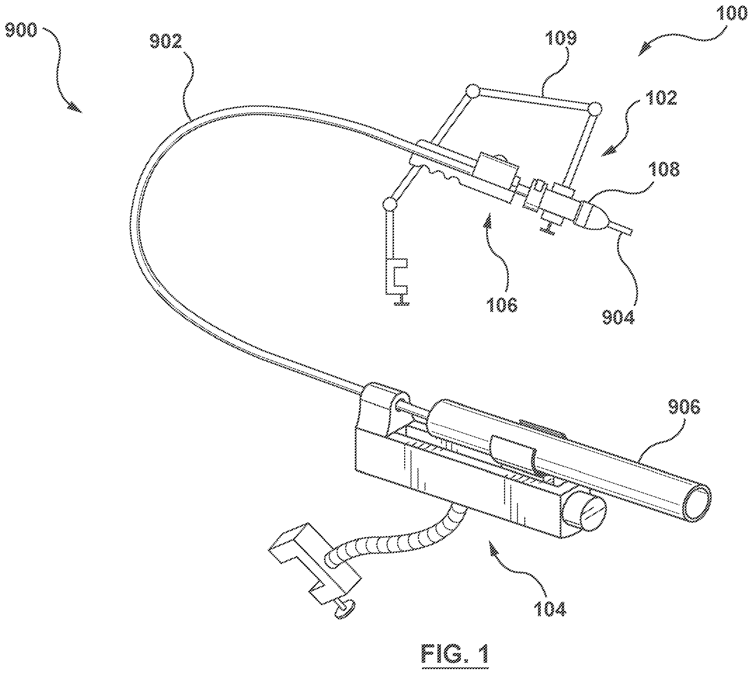

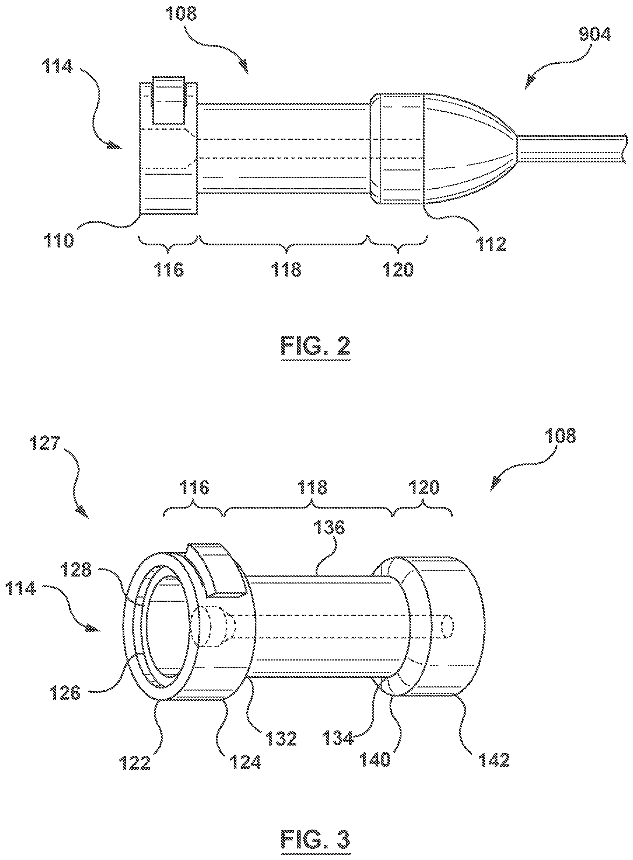

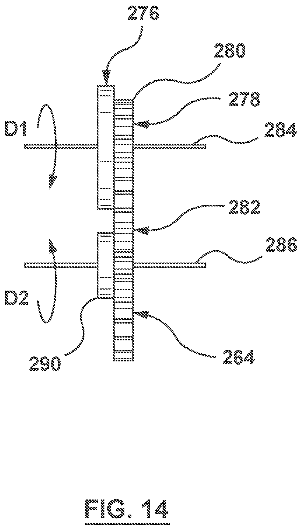

A stabilization and advancement system 100 according to an embodiment hereof is shown in FIG. 1, and in greater detail in FIGS. 2-15. In an embodiment, stabilization and advancement system 100 includes an introducer support assembly 102, a cradle mechanism 104, and an adjustment mechanism 106 for percutaneously delivering and implanting a prosthetic aortic stent valve via direct aortic transcatheter aortic valve implantation (TAVI).

In an embodiment as shown in FIG. 1 and in greater detail in FIGS. 2-15, introducer support assembly 102 includes an introducer support 108 and a flexible position clamp 109. As shown in FIG. 2, introducer support 108 includes a proximal end 110 and a distal end 112 defining a continuous lumen 114 configured to receive a shaft 902 (FIG. 1) of a delivery system 900 (FIG. 1) therein. Introducer support assembly 102 is configured to support an introducer 904 as described in greater detail below. Introducer support 108 includes a proximal portion 116, a central portion 118, and a distal portion 120 described in greater detail below.

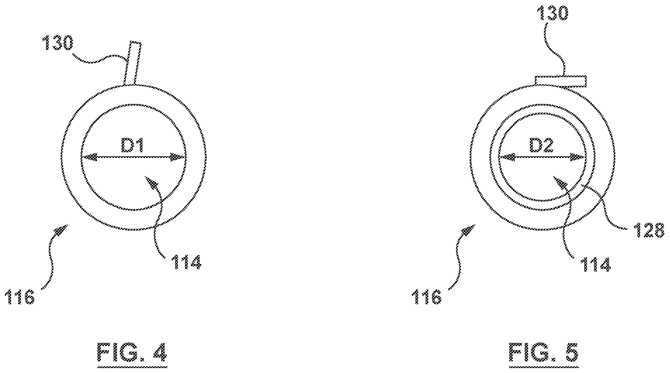

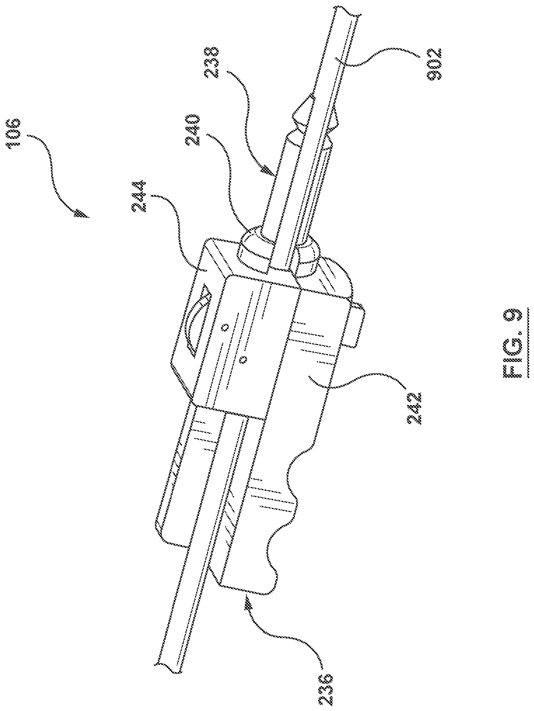

Proximal portion 116 of introducer support 108 includes a proximal end 122 and a distal end 124 defining a proximal portion of lumen 114, as shown in FIG. 3. Proximal portion 116 of housing 108 is configured to receive and couple a tube 238 (FIG. 9) of adjustment mechanism 106 (FIG. 1) therein. Proximal portion 116 further includes a lock mechanism 127. Lock mechanism 127 includes a ring channel 126 extending radially outward from and circumferentially around an inner surface of proximal portion 116 and a lock ring 128 disposed within ring channel 126. Lock ring 128 includes a first configuration wherein lock ring 128 is retracted within ring channel 126, thereby having a first inner diameter D1, as shown in FIG. 4. Lock ring 128 further includes a second configuration wherein lock ring 128 extends radially inward relative to the first configuration, as shown in FIG. 5, thereby having a second inner diameter D2. First inner diameter D1 is greater than second inner diameter D2. Moreover, lock ring 128 is configured such that lock ring 128 is fully disposed within ring channel 126 in the first configuration, and lock ring 128 is partially disposed within ring channel 126 such that an inner portion of lock ring 128 extends into lumen 114 in the second configuration. Proximal portion 116 further includes a lever 130 coupled to lock ring 128 and extending through a slot defined by proximal portion 116 for user manipulation. Lever 130 is coupled to lock ring 128 such that lever 130 includes a first configuration corresponding to the first configuration of lock ring 128, wherein lever 130 extends in a generally perpendicular direction from the outer surface of proximal portion 116, and a second configuration corresponding to the second configuration of lock ring 128, wherein lever 130 extends in a generally tangential direction from the outer surface of the proximal portion 116. Lever 130 and coupled lock ring 128 are configured for user manipulation such that lock ring 128 transitions from the first configuration to the second configuration upon manipulation of lever 130 from the first configuration to the second configuration. Lever 130 may be coupled to lock ring 128 by any means suitable for the purposes described herein.

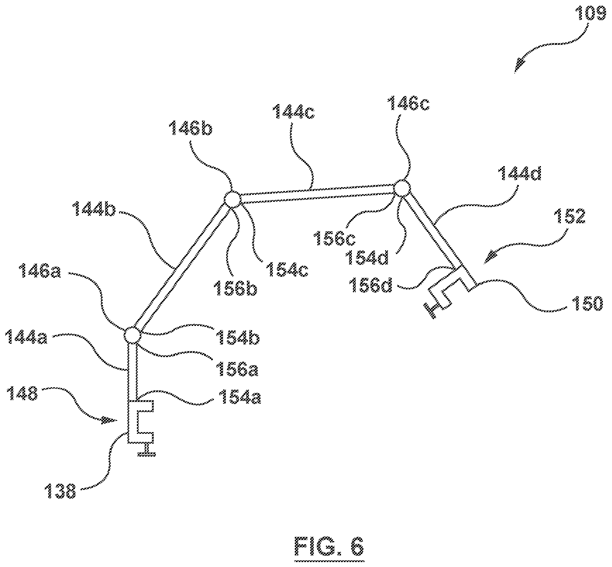

Central portion 118 of introducer support 108 includes proximal end 132 and a distal end 134 defining a central portion of lumen 114 therein, as shown in FIG. 3. Outer surface 136 of central portion 118 is configured to receive a first coupling device 138 (FIG. 6) of flexible position clamp 109 (FIG. 1) thereon such that introducer housing 108 is selectively coupled to flexible position clamp 109 (FIG. 1) as described in greater detail below.

Distal portion 120 of housing 108 includes a proximal end 140 and a distal end 142 defining a distal portion of lumen 114 therein, as shown in FIG. 3. An outer surface of distal portion 120 is configured to receive a proximal portion of an introducer 904 (FIG. 1) thereon.

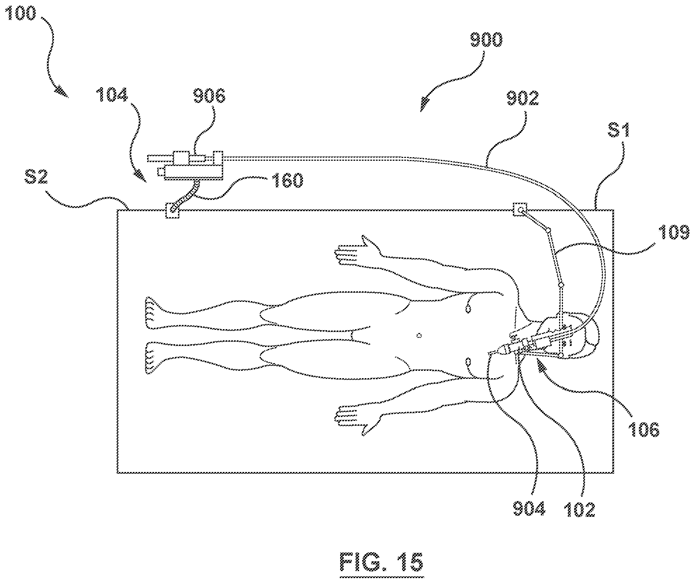

Referring to FIG. 6, in an embodiment, flexible position clamp 109 includes a plurality of arms 144a/144b/144c/144d, generally known as arms 144, and a plurality of flexible locking hinges 146a/146b/146c, generally known as hinges 146. Flexible position clamp 109 further includes first coupling device 138 at a first end 148, and a second coupling device 150 disposed at a second end 152. Flexible position clamp 109 is configured to provide an adjustable, stable platform for housing 108 of introducer support 102. Each arm 144 is a generally rod-like support structure including a first end 154 and a second end 156. Each hinge 146 is a flexible locking hinge providing movement in 3 planes and user selectable locking in a desired position. Each hinge 146 is disposed between and couples each adjacent arm 144 such that second end 156a of first arm 144a is coupled to first end 154b of second arm 144b by first hinge 146a, second end 156b of second arm 144b is coupled to first end 154c of third arm 144c by second hinge 146b, and second end 156c of third arm 144c is coupled to first end 154d of fourth arm 144d by third hinge 146c. First coupling device 138 is disposed at first end 154a of first arm 144a and configured to couple flexible position clamp 109 to housing 108. Second coupling device 150 is disposed at second end 156d of fourth arm 144d and configured to couple flexible position clamp 109 to a first stable object S1 (FIG. 15), such as an operating table, cabinet, or any other object suitable for the purposes described herein. First coupling device 138 and second coupling device 150 may be adjustable clamps, such as, but not limited to c-clamps, spring clamps, bar clamps, screw clamps, or any other coupling device suitable for the purposes described herein. While flexible position clamp 109 is described herein with four (4) arms 144 and three (3) hinges 146, this is not meant to limit the design and more or fewer arms 144 and hinges 146 may be utilized.

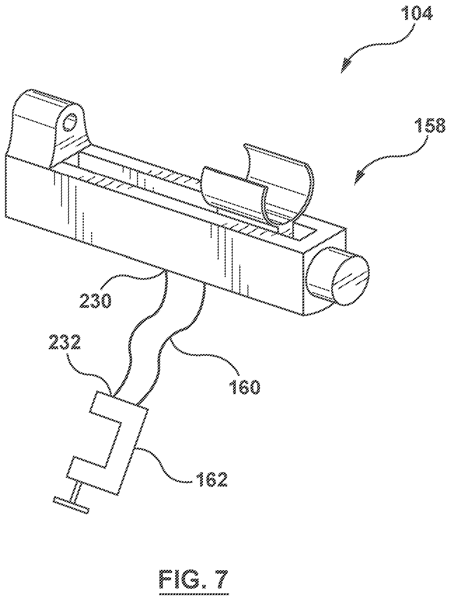

Referring to FIG. 7, in an embodiment, cradle mechanism 104 includes a cradle 158, a flexible arm 160, and a clamp 162. Cradle mechanism 104 is configured to provide stable support and positioning of a handle 906 (FIG. 1) of delivery system 900 (FIG. 1).

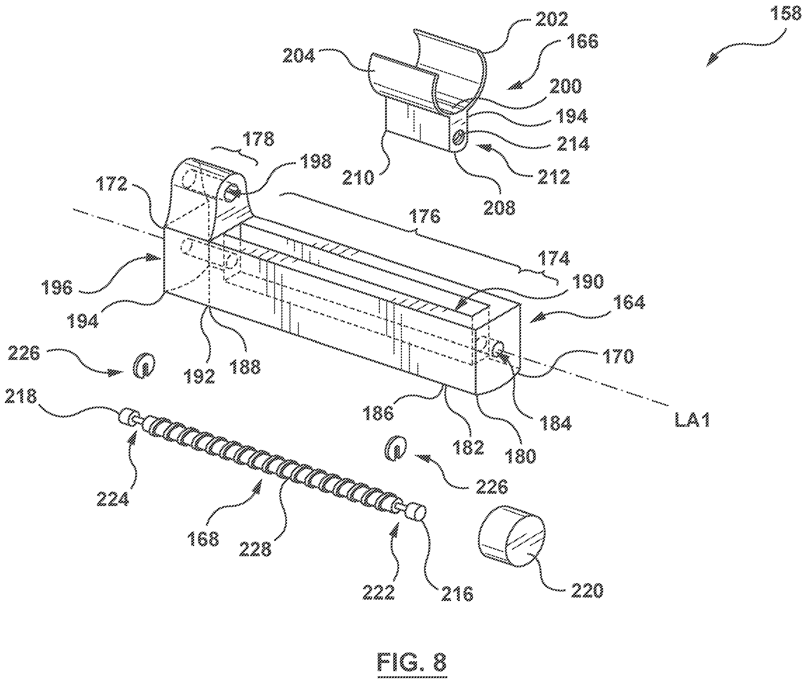

In an embodiment, cradle 158 includes a housing 164, a cradle bracket 166, and an adjustment shaft 168, as shown in FIG. 8. Cradle 158 is configured to receive handle 906 (FIG. 1) within cradle bracket 166. Cradle 158 is further configured to provide user selectable adjustment of cradle bracket 166 and handle 906 (FIG. 1) disposed therein with respect to housing 164.

Housing 164 of cradle 158 includes a proximal end 170 and a distal end 172, as shown in FIG. 8. Housing 164 further includes a proximal portion 174, a central portion 176, and a distal portion 178. Proximal portion 174 includes a proximal end 180 and a distal end 182 defining a lumen 184 configured to receive a proximal portion of adjustment shaft 168 therein. Central portion 176 includes a proximal end 186 and a distal end 188 defining an adjustment channel 190. Adjustment channel 190 is generally parallel to a first longitudinal axis LA1 of housing 164. Adjustment channel 190 extends from an upper outer surface of central portion 176 inward, towards longitudinal axis LA1 of housing 164 such that an extension 194 of cradle bracket 166 and adjustment shaft 168 may be received therein, as described in greater detail below. Distal portion 178 includes a proximal end 192 and a distal end 194 defining a lumen 196 configured to receive a proximal portion of adjustment shaft 168 therein. Distal portion 178 further defines a shaft lumen 198 configured to receive a portion of shaft 902 (FIG. 1) of delivery device 900 (FIG. 1) therein. Lumen 184, adjustment channel 190, and lumen 196 are in communication with each other, thereby forming a continuous lumen for receiving a portion of shaft 902 of delivery device 900.

Cradle bracket 166 includes a base 200, a first curved portion 202, a second curved portion 204, and extension 194, as shown in FIG. 8. First curved portion 202, second curved portion 204, and base 200 of cradle bracket 166 are configured to receive handle 906 (FIG. 1) of delivery system 900 (FIG. 1) therein. Extension 194 of cradle bracket 166 is configured to be disposed within adjustment channel 190 and move distally or proximally within adjustment channel 190 based upon user manipulation of adjustment shaft 168, as will be described in greater detail below. Extension 194 extends from a lower surface of base 200 and includes a proximal end 208 and a distal end 210 defining a lumen 212, configured to receive a portion of adjustment shaft 168 therein. Extension 194 further includes a helical thread 214 disposed on an inner surface of extension 194 in lumen 212. Helical thread 214 may be of any suitable design. Helical thread 214 may be formed for example, and not by way of limitation, as an integral portion of extension 194 or a separate unit coupled to cradle bracket 166 for example, and not by way of limitation, by fusing, welding, or other methods suitable for the purposes described herein. While helical thread 214 is shown in FIG. 8 with a specific gender, handedness, thread form, thread angle, lead, pitch, and start, this is not meant to limit the design and other configurations and combinations may be utilized.

Adjustment shaft 168 includes a proximal end 216 and a distal end 218, as shown in FIG. 8. Adjustment shaft 168 is configured to convert rotational movement of adjustment shaft 168 to longitudinal movement of cradle bracket 166 within adjustment channel 190 of housing 164 upon user manipulation, as will be described in greater detail below. Adjustment shaft 168 includes a knob 220 at proximal end 216 of adjustment shaft 168 to provide convenient manipulation/rotation of adjustment shaft 168 by a user. Adjustment shaft 168 further includes a first circumferential channel 222 disposed at a proximal portion of adjustment shaft 168 and a second circumferential channel 224 disposed at a distal portion of adjustment shaft 168. First circumferential channel 222 and second circumferential channel 224 are each configured to receive a retainer 226 to retain shaft 168 within lumen 186, adjustment channel 190, and lumen 196, and to prevent longitudinal movement (proximally or distally) of shaft 168 with respect to housing 164. Adjustment shaft 168 further includes a helical thread 228 disposed on outer surface of adjustment shaft 168, Helical thread 228 of adjustment shaft 168 is configured to correspond to and engage helical thread 214 of cradle bracket 166 when received therein. Helical thread 228 may be of any suitable design corresponding to helical thread 214 of cradle bracket 166. Helical thread 228 may be formed for example, and not by way of limitation, as an integral portion of shaft 168 or a separate unit coupled to shaft 168 by, for example, and not by way of limitation, adhesives, welding, or other methods suitable for the purposes described herein. While helical thread 228 is shown in FIG. 8 with a specific gender, handedness, thread form, thread angle, lead, pitch, and start, this is not meant to limit the design and other configurations and combinations maybe utilize.

Knob 220 may be formed for example, and not by way of limitation, as an integral portion of adjustment shaft 168 or a separate unit coupled to adjustment shaft 168 by, for example and not by way of limitation, adhesive, welding, setscrew, or other methods suitable for the purposes described herein. Knob 220 may include indentations or other surface modifications on an outer radius thereof to assist the user in manipulation/rotation of adjustment shaft 168. While knob 220 is described herein as being circular in shape, this is not meant to limit the design, and other configurations of knob 220 may be utilized.

When cradle mechanism 104 is assembled, extension 194 of cradle bracket 166 resides within adjustment channel 190 of the housing 164, as shown in FIG. 7. Adjustment shaft 168 is retained within lumen 184 of proximal portion 174 and lumen 196 of distal portion 178 of housing 164 by retainers 226 disposed within first circumferential channel 222 and second circumferential channel 224 of adjustment shaft 168. Moreover, adjustment shaft 168 is received within lumen 212 of extension 194 disposed within adjustment channel 190 of housing 164 such that helical thread 214 of extension 194 engages corresponding helical thread 228 of adjustment shaft 168. With cradle mechanism 104 so assembled, user manipulation/rotation of knob 220 rotates adjustment shaft 168. As adjustment shaft 168 cannot travel longitudinally (proximally or distally) due to retainers 226, helical thread 228 of adjustment shaft 168 engaged with corresponding helical thread 214 of extension 194 moves cradle bracket 166 longitudinally (proximally or distally) within adjustment channel 190 of housing 164. The direction of longitudinal movement (proximal or distal) is based upon rotational direction of knob 220 and configuration of helical thread 228 and corresponding helical thread 214.

Referring back to FIG. 7, in an embodiment, flexible arm 160 includes a first end 230 coupled to a surface of housing 164 and a second end 232 coupled to clamp 162, Flexible arm 160 is configured to provide an adjustable, stable platform for cradle mechanism 104 and handle 906 (FIG. 1) of delivery system 900 (FIG. 1) disposed therein. Flexible arm 160 may include a ball-and-socket configuration, a flexible chain configuration, or any other flexible arm configuration suitable for the purposes described herein. Clamp 162 is disposed at second end 232 of flexible arm 160 and is configured to couple flexible arm 160 to a second stable object S2 (FIG. 15), such as an operating table, cabinet, or any other object suitable for the purposes described herein. Clamp 162 may be, for example and not byway of limitation, a c-clamp, spring clamp, bar clamp, screw clamp, or any other coupling device suitable for the purposes described herein. While clamp 162 is described herein as coupling flexible arm 160 to second stable object S2, second stable object S2 may be the same object as first stable object S1 or a different object than first stable object S1, previously described with respect to flexible position clamp 109 (FIG. 6) of introducer support assembly 102.

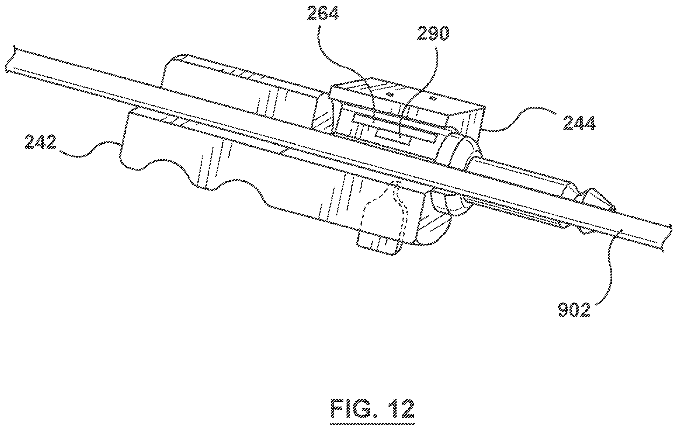

Referring to FIGS. 9-14, in an embodiment, adjustment mechanism 106 includes a housing 236 and a tube 238 coupled to a distal end 240 of housing 236. Adjustment mechanism 106 includes a first configuration as shown in FIG. 9 and a second configuration as shown in FIG. 12, and described in greater detail below. The first configuration may also be referred to as the closed configuration or the operating configuration, while the second configuration may be referred to as the open configuration. In general, adjustment mechanism 106 is configured to provide convenient and accurate advancement and retraction of shaft 902 disposed therein, as described in greater detail below. Additionally, adjustment mechanism 106 is configured to be disposed proximal of introducer support 108 (FIG. 1) of introducer support assembly 102. More specifically, tube 238 of housing 236 is configured to be disposed within proximal portion 116 (FIG. 2) of introducer support 108 (FIG. 2) and coupled thereto.

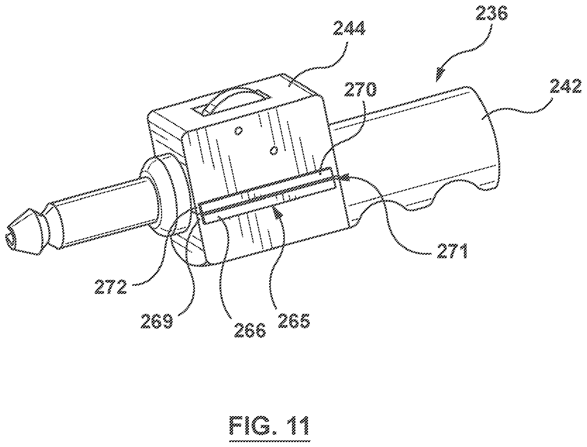

Housing 236 of adjustment mechanism 106 is generally cylindrical and includes a base 242 and an adjustment portion 244 coupled to base 242 by a hinge 265 (FIG. 11). Base 242 of housing 236 is configured to provide convenient grasping and handling by the user. Base 242 and adjustment portion 244 are configured such that base 242 and adjustment portion 244 work together to provide precise distal and proximal movement of shaft 902 disposed therein, as described in greater detail below.

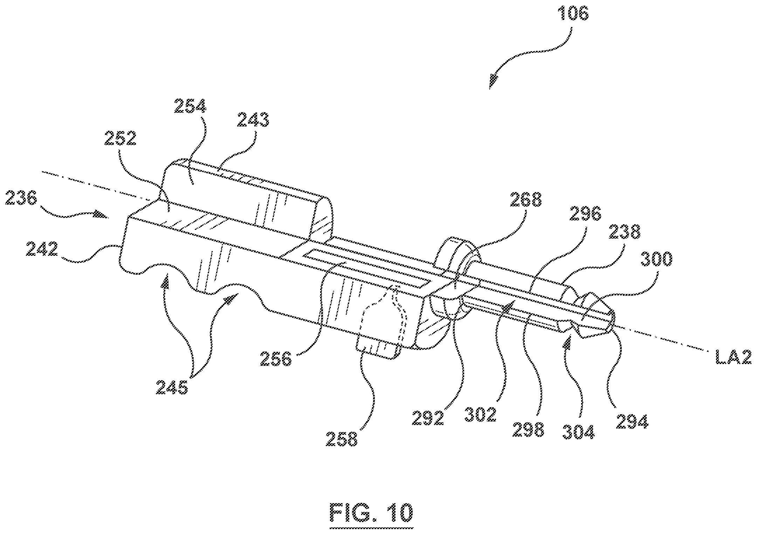

In an embodiment shown in FIG. 10, base 242 of adjustment mechanism 106 defines a first surface 252 and a shoulder 243 within a proximal portion of base 242. First surface 252 is a generally horizontal surface on the interior of base 242 and is generally aligned with a longitudinal axis LA2 of adjustment mechanism 106. First surface 252 is configured to abut shaft 902 (FIG. 9) positioned thereon. Shoulder 243 includes a second surface 254. Second surface 254 is a generally vertical surface of shoulder 243 disposed along and extending vertically upward from a left edge (when viewed from a position proximal of adjustment mechanism 106) of first surface 252. First surface 252 and second surface 254 are configured such that a lower surface of shaft 902 (FIG. 9) disposed therein abuts first surface 252 and a left side surface of shaft 902 abuts second surface 254, (when viewed from a position proximal of adjustment mechanism 106). Stated another way, with shaft 902 disposed within adjustment mechanism 106, shaft 902 is cradled on a lower side by first surface 252 and on a left side by second surface 254,

Base 242 includes indentations 245 on an outer surface of the lower portion of base 242 to assist the user in grasping and manipulation of housing 236. While FIGS. 9-11 show base 242 with two (2) indentations 245, this is not meant to limit the design and more or fewer indentations 245 and/or other surface modification to enhance user comfort and control may be utilized.

Base 242 further includes a third surface 256 within a central portion of base 242, as shown in FIG. 10. Third surface 256 is coupled to an actuator 258 within a cavity defined by base 242. Actuator 258 extends through a slot defined by base 242. Third surface 256 and coupled actuator 258 are configured to provide a user-selectable frictional surface for precise distal and proximal movement of shaft 902 (FIG. 9) disposed therein, as described in greater detail below. Third surface 256 is a generally horizontal surface, aligned with second longitudinal axis LA2 and positioned distally of first surface 252. Third surface 256 includes a first configuration wherein third surface 256 does not provide frictional engagement with shaft 902 disposed therein, and a second configuration wherein third surface 256 provide frictional engagement with shaft 902 disposed therein. Actuator 258 is configured such that when not manipulated by the user, third surface 256 is in the first configuration, and when manipulated by the user, third surface 256 transitions to the second configuration. Upon release of actuator 258, third surface 256 transitions from the second configuration back to the first configuration. While the embodiment described herein includes third surface 256 biased to the first configuration, this is not meant to limit design, and other configurations may be utilized including, but not limited to third surface 256 biased to the second configuration, having no bias, or any other configuration suitable for the purposes described herein.

In the embodiment shown in FIG. 10, base 242 further defines a fourth surface 268 within a distal portion of base 242. Fourth surface 268 is a generally horizontal surface on the interior of base 242 of housing 236, and is generally aligned with a longitudinal axis LA2 and first surface 252 of adjustment mechanism 106, and is disposed distal of third surface 256. Fourth surface 268 is configured to abut shaft 902 (FIG. 9) positioned thereon.

In an embodiment shown in FIG. 11, adjustment portion 244 is coupled to base 242 by a hinge 265. Hinge 265 is a barrel hinge as is well known in the art. Hinge 265 includes a first barrel 266 at a first edge 269 of base 242, a corresponding second barrel 270 disposed at a second edge 272 of adjustment portion 244, and a pivot 271. First barrel 266 is coupled to corresponding second barrel 270 by pivot 271. Hinge 265 is configured to allow user selectable pivoting of adjustment portion 244 away from base 242 to accept or remove shaft 902 therein, as shown in FIG. 12, and to pivot over base 242 to retain and manipulate shaft 902 disposed therein, as shown in FIG. 9. Hinge 265 is described herein as a barrel hinge, but this is not meant to limit the design and other configurations of hinge 265 may be utilized suitable for the purposes described herein. Moreover, while hinge 265 is described herein as a separate component from base 242 and adjustment portion 244, hinge 265 may be formed as an integral portion of base 242 and adjustment mechanism 244. Hinge 265 may be coupled to base 242 and adjustment portion 244 for example, and not by limitation, by adhesives, fusing, welding, or any other method suitable for the purposes described herein.

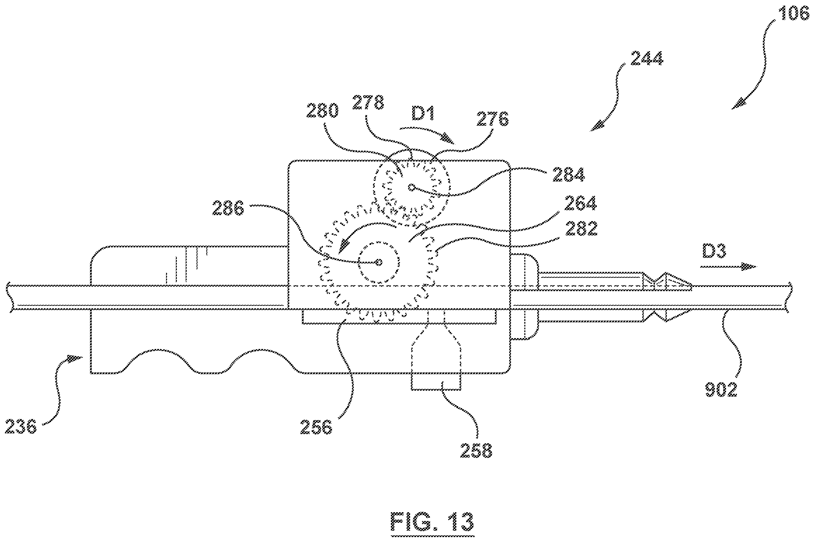

Referring to FIGS. 13-14, adjustment portion 244 includes a thumbwheel 276 and a wheel 264 disposed within a cavity of adjustment portion 244. Thumbwheel 276 includes first teeth 278 disposed on an outer radius of a first shoulder 280. Wheel 264 includes second teeth 282 disposed on an outer radius of wheel 264. Thumbwheel 276 is disposed within adjustment portion 244 on a first pivot axis 284 such that wheel 264 rotates about first pivot axis 284. The upper radius of thumbwheel 276 extends through a longitudinal slot defined by adjustment portion 244 of housing 236 for user manipulation. Wheel 264 is disposed within adjustment portion 244 on a second pivot axis 286 such that wheel 264 rotates about second pivot axis 286. Thumbwheel 276 and wheel 264 are configured such that first teeth 278 of thumbwheel 276 engage with corresponding second teeth 282 of wheel 264 as described in greater detail below. While FIGS. 13-14 show adjustment portion 244 having a specific number of first teeth 278 on thumbwheel 276 and a specific number of corresponding second teeth 282 on wheel 264, this is not meant to limit the design and the various numbers of first teeth 278 and corresponding second teeth 282 may be utilized.

Adjustment portion 244 is generally constructed to provide selective retraction/advancement of shaft 902 of delivery system 900 relative to introducer 904 as shown in FIG. 1. Adjustment portion 244 can have a variety of constructions and/or devices capable of providing the desired user interface and the current embodiment shown in FIGS. 9-14 is not meant to limit the design, but rather provide an example of one possible embodiment.

With adjustment mechanism 106 in the first configuration, an outer radius of a second shoulder 290 of wheel 264 contacts an outer surface of shaft 902, as shown in FIG. 9 and FIG. 13. Adjustment portion 244 of adjustment mechanism may be pivoted along hinge 265 to the second configuration such that second shoulder 290 of wheel 264 does not contact the outer surface of shaft 902. With adjustment mechanism in the second (open) configuration, adjustment mechanism 106 may be disposed over or removed from shaft 902, as shown in FIG. 12. Stated another way, adjustment portion 244 of adjustment mechanism 106 is configured to pivot away from base 242 to accept or remove shaft 902 therein and to pivot over base 242 to retain and manipulate shaft 902 therein.

Referring back to FIG. 10, tube 238 of adjustment mechanism 106 is includes a proximal end 292 and a distal end 294. Tube 238 further includes a first edge 296 and a second edge 298. A fifth surface 300 is disposed between first edge 296 and second edge 298 in a first circumferential direction. A gap or opening 302 is defined between first edge 296 and second edge 298 in a second circumferential direction opposite the first circumferential direction. Fifth surface 300 and opening 302 also extend between proximal end 292 and distal end 294 of tube 238 such that tube 238 is not a complete tube circumferentially. Fifth surface 300 is configured to abut shaft 902 when shaft 902 is disposed within tube 238. Opening 302 enables shaft 902 to be inserted therethrough such that adjustment mechanism 106 may be placed over or removed from shaft 902 without adjustment mechanism 106 needing to be slid to a proximal or distal end of shaft 902. Thus, adjustment mechanism 902 may be placed over or removed from shaft 902 at any location along shaft 902. Tube 238 further Includes a circumferential channel 304 at a distal portion of tube 238, extending from an outer surface of distal portion 306 inward, and extending circumferentially from first edge 296 to second edge 298, Circumferential channel 304 is configured to receive a portion of lock ring 128 (FIG. 5) of proximal portion 116 of introducer support 102 therein when lock ring 128 is in the second configuration. Stated another way, when tube 238 is disposed within proximal portion 116 of introducer support 102, tube 238 of adjustment mechanism 106 may be coupled thereto by lock ring 128 in the second configuration. Proximal end 292 of tube 238 is coupled to distal end 240 of housing 236 such that lumen 114 (FIG. 2) of introducer support 102 (FIG. 1) aligns longitudinally with fifth surface 300 of tube 238 such that shaft 902 of delivery system 900 may extend distally from handle 906, through adjustment mechanism 106 and introducer support 102 and on through introducer 904, as shown in in FIG. 1. While tube 238 is described herein as a separate component of adjustment mechanism 106, tube 238 may simply be an extension of housing 236. Tube 238 maybe coupled to housing 236 for example, and not by way of limitation, by adhesives, welding, fusing, or other methods suitable for the purposes described herein.

With the above understanding of components of adjustment mechanism 106 in mind, operation and interaction of components of the present embodiment may be explained. FIG. 13 illustrates adjustment mechanism 106 in the first configuration and actuator 258 manipulated by the user such that third surface 256 is in the second configuration. Adjustment mechanism 106 is configured such that, as thumbwheel 276 is rotated in a first direction D1, first teeth 278 of first shoulder 280 of thumbwheel 276 engage with corresponding second teeth 282 of the outer radius of wheel 264 to rotate of wheel 264 in a second direction D2 opposite first direction D1. Moreover, with actuator 258 actuated, third surface 256 is positioned against shaft 902 such that shaft 902 is frictionally engaged between the outer radius of second shoulder 290 of wheel 264 and third surface 256. Thus, rotation of wheel 264 in the second direction D2, and thus rotation of second shoulder 290 is direction D2 causes movement of shaft 902 in a third direction D3 (distally in this example). Movement of shaft 902 in third direction D3 is relative to introducer support 108 and adjustment mechanism 106. Stated another way, with actuator 258 manipulated by the user and adjustment portion 244 disposed over shaft 902, as thumbwheel 276 is rotated in direction D1, shaft 902 moves distally. Similarly, rotation of thumbwheel 276 in direction D2 causes shaft 902 to move proximally.

As described above, actuator 258 is actuated to provide frictional engagement between third surface 256 and shaft 902. However, in other embodiments, actuator 258 is not required. For example, and not by way of limitation, adjustment mechanism 106 may be configured such that closing of actuator portion 244 over shaft 902 provides sufficient frictional engagement second shoulder 290 shaft 902 and a surface of adjustment mechanism 106 such that rotation of second shoulder 290 causes shaft 902 to advance or retract.

Further, adjustment mechanism 108 is described with thumbwheel 276 and wheel 264. However, more or fewer wheels/gears may be utilized. For example, and not by way of limitation, first shoulder 280 may be directly frictionally engaged with shaft 902 such that wheel 264 and second shoulder 290 are not required.

With the above understanding of components of stabilization and advancement system 100 in mind, operation and interaction of components of the present disclosure may be explained. Referring to FIG. 15, flexible position clamp 109 of introducer support assembly 102 is coupled to first stable object S1 (operating table) near the right shoulder of a patient. Cradle mechanism 104 is coupled to second stable object S2 (operating table) near the right foot of the patient. Positioning of the components as described provides a familiar and similar configuration to that of a trans-femoral procedure and reduces exposure of treating clinicians to radiation extended exposure from fluoroscopy. Introducer 904 is coupled to introducer support 108. Using procedures for direct aortic TAVI, introducer 904 is disposed within the patient at a desired access site for access to the patient's aorta. Flexible position clamp 109 is manipulated by the user to a desired position and coupled to introducer support 108, providing stability and support for introducer 904. Shaft 902 of delivery system 900 is advanced through introducer support 102 and introducer 904 into patient's vasculature. Handle 906 of delivery system 900 is disposed within cradle mechanism 104, and flexible arm 160 of cradle mechanism 104 is adjusted to a desired position.

Adjustment mechanism 106 with adjustment portion 244 in the second (open) configuration, is positioned over shaft 902 such that shaft 902 is disposed on and abuts first surface 252, second surface 254, fourth surface 268, and fifth surface 300 of adjustment mechanism 106. With lever 130 of introducer support 102 in the first configuration (FIG. 4) such that lock ring 128 is in the first configuration, adjustment mechanism 106 is advanced distally such that the distal portion of tube 238 is disposed within lumen 114 of proximal portion 116 of introducer support 108. Lever 130 is manipulated to the second configuration (FIG. 5) such that lock ring 128 transitions to the second configuration and couples adjustment mechanism 106 to introducer support 108. Adjustment portion 244 of adjustment mechanism 106 is pivoted to the first (closed) configuration to retain shaft 902 therein. Adjustment mechanism 106 is now supported and stabilized by introducer support 108.

Delivery system 900, so disposed and stabilized by stabilization and advancement system 100 requires fewer users than non-stabilized direct aortic TAVI. More specifically, current delivery systems, such as exemplary delivery system 900, are designed to be utilized for procedures conducted at a variety of access points including, but not limited to femoral access and direct aortic access. Thus, current delivery systems are of a length suitable for the longest possible access path, femoral access. However, when a current delivery system is utilized for direct aortic access, a much shorter access path than with femoral access, a majority of the delivery system is disposed external to the patient during a procedure. This external portion of the delivery system is currently held in place and stabilized by a second user. With the stabilization and advancement system 100 of FIG. 15, the external portion of the delivery system 900 is held in place by the stabilization and advancement system 100, thus eliminating the requirement for the second user.

With the delivery system 900 supported by the stabilization and advancement system 100, the user may rotate thumbwheel 276 with actuator 258 actuated (if necessary) to accurately advance or retract shaft 902 and components disposed therein. Should a bail-out be required during the procedure, adjustment mechanism 106 may quickly be disconnected from delivery system 900 by manipulating lever 130 (FIG. 4) of introducer support 102 to the first configuration, thereby releasing adjustment mechanism 106 from introducer support 108, retracting adjustment mechanism 106 proximally from introducer support 108, manipulating adjustment portion 244 of adjustment mechanism 108 to the second (open) configuration, and removing adjustment mechanism 106 from shaft 902 by passing shaft 902 through opening 302 of tube 238.

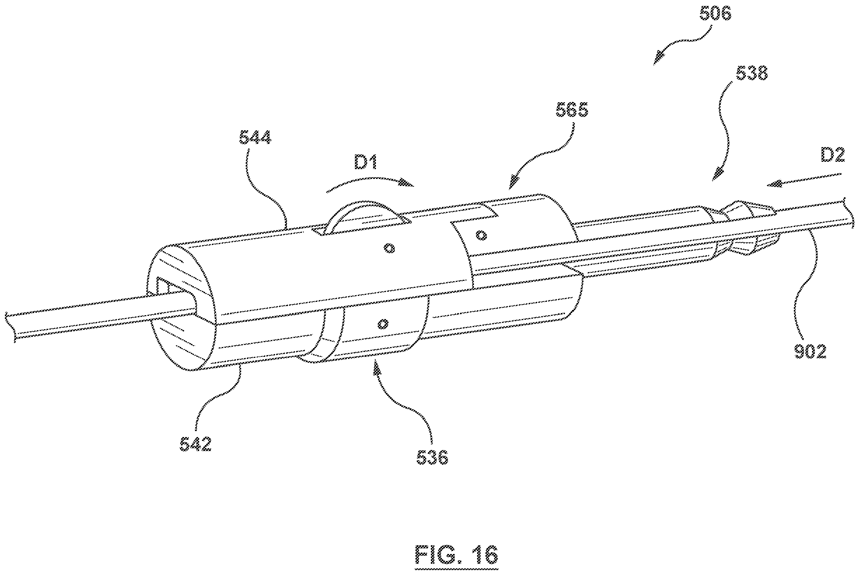

Referring to FIGS. 16-19, an adjustment mechanism 506 according to another embodiment hereof includes a housing 536 and a tube 538 disposed at a distal end 540 of housing 536. Housing 536 of adjustment mechanism 506 includes a base 542 and an adjustment portion 544 coupled to base 542 by a hinge 565. Tube 538 is the same as tube 238 described above with respect to adjustment mechanism 106, and thus will not be described with respect to adjustment mechanism 506.

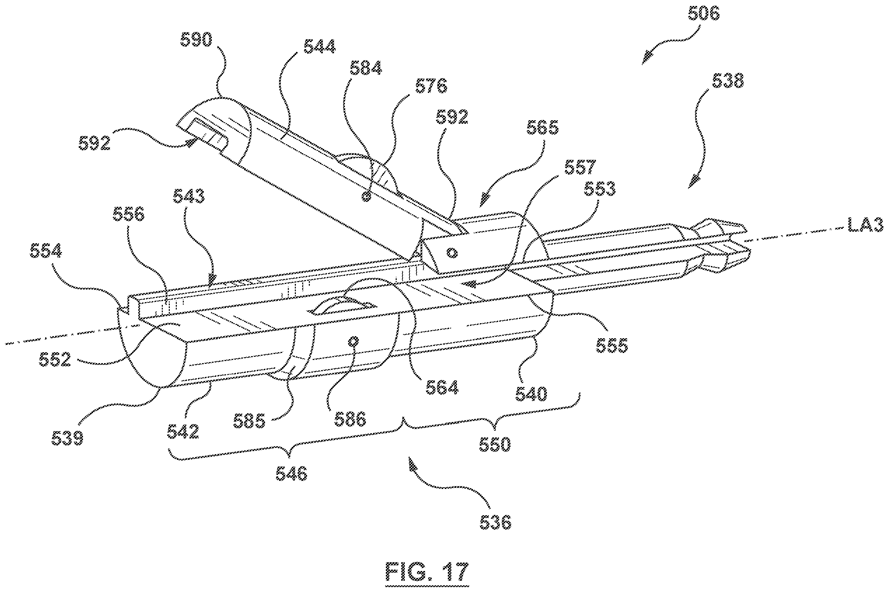

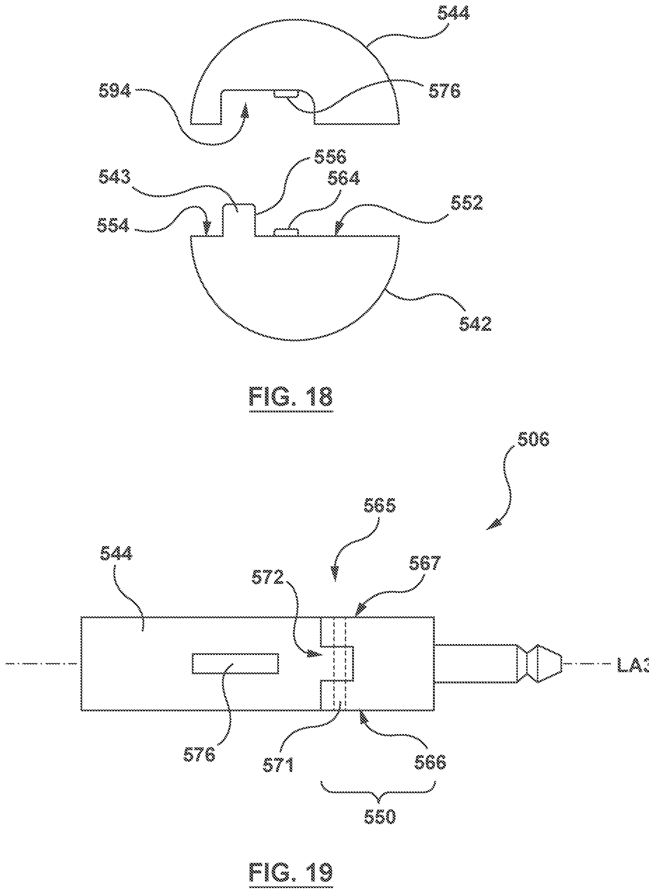

Base 542 includes a proximal end 539 and distal end 540 defining a first surface 552 extending from proximal end 539 to distal end 540, as shown in FIGS. 17-18. First surface 552 is a generally horizontal surface on the interior of housing 536, as shown in FIG. 18, and is generally aligned with a longitudinal axis LA3 of base 542. First surface 552 is configured to abut shaft 902 of delivery system 900 positioned thereon, as shown in FIG. 16, Base 542 further includes a proximal portion 546 and a distal portion 550, as shown in FIG. 17. Proximal portion 546 is a generally half cylindrical shape (a cylinder cut longitudinally), with first surface 552 defined opposite the curved portion of the half cylinder. In other words first surface 552 is the flat surface of the half cylinder. In an embodiment, first surface 552 may be offset laterally towards one side of proximal portion 546 (right when viewed from a proximal portion of adjustment mechanism 506). While the outer radius of proximal portion 546 is shown in FIG. 17 with a smooth exterior surface, this is not meant to limit the design, and the outer surface of proximal portion 546 may include indentations or other surface modifications on an outer radius thereof to assist the user in manipulation of adjustment mechanism 506.

Proximal portion 546 defines a second surface 554 similar to first surface 552 laterally offset toward an opposite side proximal portion 546 (left when viewed from a position proximal of adjustment mechanism 506), as shown in FIGS. 17-18. Second surface 554 is a generally horizontal surface on the interior of proximal portion 546, generally parallel with longitudinal axis LA3 of base 542 of housing 536, As with first surface 552, second surface 554 is generally the flat surface of the half cylinder shape of proximal portion 546 of base 542.

Proximal portion 546 further defines a shoulder 543 including a third surface 556 disposed between first surface 552 and second surface 554, as shown in FIGS. 17-18. Third surface 556 is generally perpendicular to first and second surfaces 552, 554. Shoulder 543 extends generally perpendicularly away from first and second surfaces 552, 544 in a direction opposite the curved portion of the half cylinder (upwards in FIGS. 17). Shoulder 543 is also disposed between first surface 552 and second surface 554. First surface 552 and third surface 556 are configured such that a lower surface of shaft 902 disposed therein abuts an upper surface of first surface 552 and a left side surface of shaft 902 abuts a right-side surface of third surface 556, when viewed from a position proximal of adjustment mechanism 506. Stated another way, with shaft S02 disposed within adjustment mechanism 506, shaft 902 is cradled on a lower side by first surface 552 and on a left side by third surface 556. Second surface 554 is configured to abut a corresponding lower surface of adjustment portion 544, as described in greater detail below.

Proximal portion 546 further includes a wheel 564 disposed within a cavity of base 542, as shown in FIG. 17. In the embodiment shown, base 542 includes a bulge 585 to accommodate wheel 564, but such a bulge is not required. Wheel 564 extends through a slot defined by first surface 552 such that an outer radius of wheel 564 is slightly above first surface 552. Wheel 564 is disposed within proximal portion 546 of base 536 on a second pivot axis 586 such that wheel 564 rotates about second pivot axis 584. Wheel 564 is configured to provide a frictional surface for precise distal and proximal movement of shaft 902 disposed therein, as described in greater detail below.

Distal portion 550 of base 542 is generally cylindrical in shape and defines a distal portion of first surface 552, as shown in FIG. 17. Distal portion 550 includes a first edge 553 and a second edge 555 along an outer surface of distal portion 550 defining an opening 557 between first edge 553 and second edge 554. First surface 552 is part of a surface extending from first edge 553 to second edge 554 in a circumferential direction opposite opening 557 such that distal portion 550 is essentially not a complete tube circumferentially. Opening 557 is configured to receive shaft 902 therethrough.

In an embodiment shown in FIG. 19, hinge 565 is a barrel hinge. Hinge 565 includes a first barrel 566 and a second barrel 567 at a proximal portion of distal portion 550 of base 542. Hinge 565 further includes a corresponding third barrel 572 at a distal portion of adjustment portion 544, and a pivot 571. First barrel 566 and second barrel 567 are coupled to corresponding third barrel 572 by pivot 571 disposed in generally transverse to longitudinal axis LA3. Hinge 565 is configured to allow user selectable pivoting of adjustment portion 544 away from base 542 to accept or remove shaft 902 therein, as shown in FIG. 17, and towards base 542 to retain and manipulate shaft 902 disposed therein, as shown in FIG. 16. Hinge 565 is shown in FIGS. 16-19 as a barrel hinge, but this is not meant to limit the design and other configurations of hinge 565 may be utilized suitable for the purposes described herein. Moreover, while hinge 565 is described herein as an integral component of base 542 and adjustment portion 544, hinge 565 may be formed as a separate component coupled to base 542 and adjustment mechanism 544.

In an embodiment, adjustment portion 544 is a generally half cylindrical shape and includes a proximal end 590 and a distal end 592, A channel 594 is defined within the flat surface of the half-cylinder opposite the rounded surface, as shown in FIGS. 17-18. Channel 594 is configured to receive shoulder 543 of base 546 and shaft 902 therein when adjustment mechanism 506 is in a first configuration as described in greater detail below.

Adjustment portion 544 includes third barrel 572 of hinge 565 defined by adjustment portion 544, and a thumbwheel 576, as shown in FIG. 17. Thumbwheel 576 is disposed in a cavity within adjustment portion 544 on a pivot axis 584 such that thumbwheel 576 rotates about pivot axis 584, as shown in FIG. 17. The upper radius of thumbwheel 576 extends through a longitudinal slot defined by adjustment portion 544 of housing 536 for user manipulation.

Adjustment portion 544 is generally constructed to provide selective retraction/advancement of a shaft 902 (FIG. 16) of delivery system 900 relative to introducer 904. Adjustment portion 544 can have a variety of constructions and/or devices capable of providing the desired user interface and the current embodiment shown in FIGS. 16-19 is not meant to limit the design, but rather provide an example of one possible embodiment.

Referring back to FIG. 16, in an embodiment, in the first configuration of adjustment mechanism 506, adjustment portion 544 is pivoted towards base 542 such that the outer radius of thumbwheel 576 contacts an outer surface of shaft 902, and the outer radius of wheel 564 contacts an outer surface of shaft 902 opposite where thumbwheel 576 contact shaft 902. FIG. 17 shows a second (open) configuration of adjustment mechanism 506, wherein adjustment portion 544 is pivoted away from base 536 such that the outer radius of thumbwheel 576 does not contact the outer surface of shaft 902. In the second configuration, adjustment mechanism 506 may be disposed over or removed from shaft 902. Stated another way, adjustment portion 544 of adjustment mechanism 506 is configured to pivot away from base 542 to accept or remove shaft 902 therein and to pivot over base 542 to retain and manipulate shaft 902 therein.

With the above understanding of components of adjustment mechanism 506 in mind, operation and interaction of components of the present embodiment may be explained. FIG. 16 illustrates adjustment mechanism 506 in the first configuration with the shaft 902 disposed therein. In the first configuration, as thumbwheel 576 is rotated in a first direction D1, the outer radius of thumbwheel 576 engages shaft 902 of delivery system 900 such that rotation of thumbwheel 576 in first direction D1 frictionally moves shaft 902 in a second direction D2. Movement of shaft 902 in direction D2 is relative to an introducer support 102 (not shown in FIGS. 16-19) and adjustment mechanism 506 of the stability and adjustment mechanism. In the embodiment shown, directions D1 and D2 are opposite in the sense that pushing the exposed portion of thumbwheel 576 towards the distal end results in the shaft moving proximally. However, this is not meant to limit the design and gearing or other devices may be included such that pushing the exposed portion of thumbwheel 576 towards the distal end results in shaft 902 moving distally.

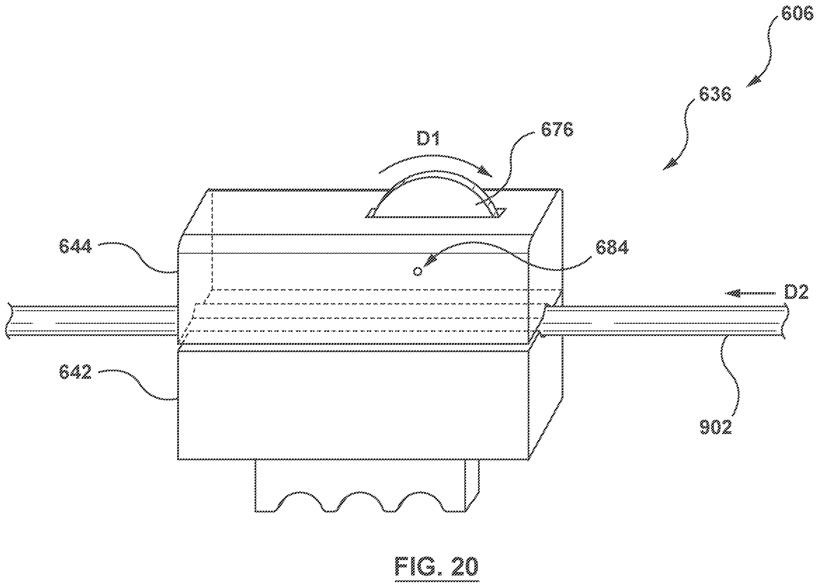

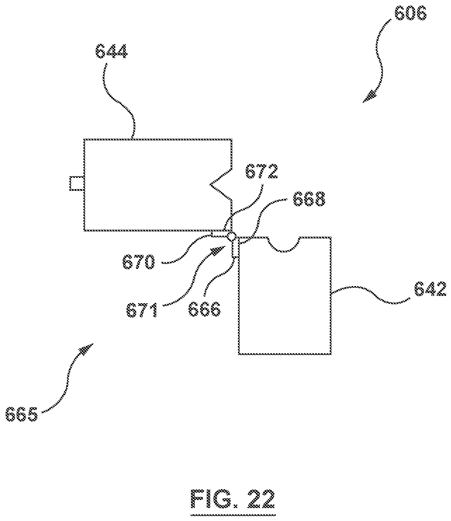

FIGS. 20-22 show another embodiment an adjustment mechanism 606. Adjustment mechanism 606 includes a housing 636. Housing 636 of adjustment mechanism 606 is generally rectangular and includes a base 642 and an adjustment portion 644 coupled to base 642 by a hinge 665 (FIG. 22).

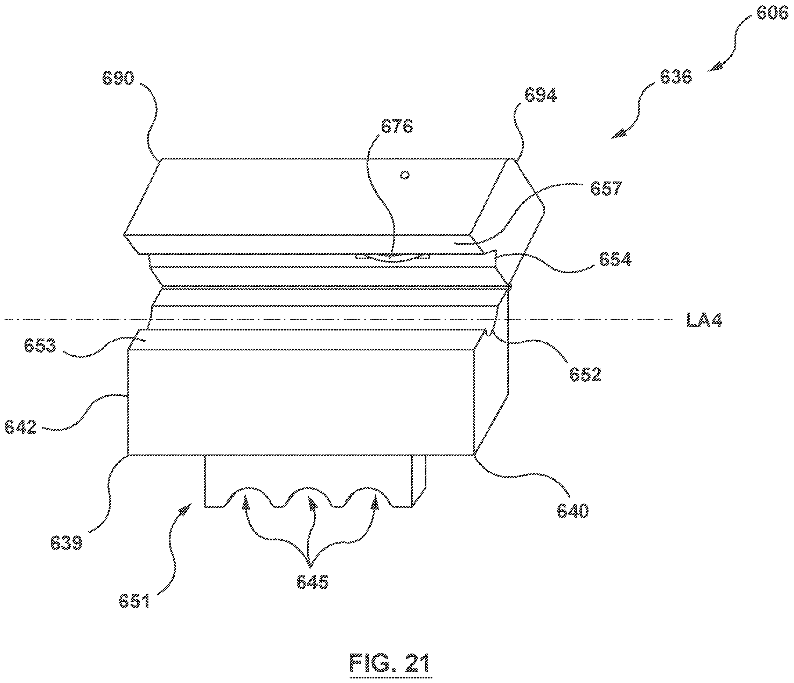

Referring to FIG. 21, base 642 includes a proximal end 639 and a distal end 640 defining a first channel 652 extending from proximal end 639 to distal end 640. First channel 652 is a generally longitudinal channel on the interior of base 642 of housing 636, generally aligned with a longitudinal axis LA of housing 636. First channel 652 is configured to receive a portion of shaft 902 of delivery system 900 therein. While first channel 652 is shown in FIGS. 20-22 as a semi-circular channel, this is not meant to limit the design, and other configurations may be utilize including, but not limited to, v-channels, square channels, or any other configuration suitable for the purposes described herein. Base 642 is generally rectangular, wherein first channel 652 is disposed in a surface 653 of base 642 facing a corresponding surface of adjustment portion 644. An outer surface 655 of base 642 opposite surface 653 may include an extension 651 defining a plurality of indentations 645 for ease of handling. However, this is not meant to limit the design, and other configurations may be utilized including, but not limited to having no indentations or extension, a different quantity of indentations, indentations defined by outer surface 655, or any other configuration suitable for the purposes described herein.

In an embodiment shown in FIG. 22, adjustment portion 644 is coupled to base 642 by a hinge 665. Hinge 665 may be a barrel hinge. Hinge 665 includes a first barrel 666 at a first edge 668 of base 642, a corresponding second barrel 670 disposed at a second edge 672 of adjustment portion 644, and a pivot 671. First barrel 666 is coupled to corresponding second barrel 670 by pivot 671. Hinge 665 is configured to allow user selectable pivoting of adjustment portion 644 away from base 642 to accept or remove shaft 902 therein, as shown in FIGS. 21-22, and to pivot adjustment portion 644 over base 642 to retain and manipulate shaft 902 disposed therein, as shown in FIG. 20. Hinge 665 is described herein as a barrel hinge, but this is not meant to limit the design and other configurations of hinge 665 may be utilized suitable for the purposes described herein. Moreover, while hinge 665 is described herein as a separate component from base 642 and adjustment portion 644, hinge 665 may be formed as an integral portion of base 642 and adjustment mechanism 644. Hinge 665 may be coupled to base 642 and adjustment portion 644 for example, and not by limitation, by adhesives, fusing, welding, or any other method suitable for the purposes described herein.

In an embodiment, adjustment portion 644 includes a proximal end 690 and a distal end 692 defining a second channel 654 extending from proximal end 690 to distal end 692, as shown in FIG. 21. Second channel 654 is a generally longitudinal channel in a surface 657 of adjustment portion 644, generally aligned with longitudinal axis LA of housing 636 when adjustment portion 644 is in the first (closed) configuration. Second channel 654 is configured to receive a portion of shaft 902 (FIG. 20) therein. While second channel 654 is shown in FIGS. 20-22 as a v-channel, this is not meant to limit the design, and other configurations may be utilize including, but not limited to, semicircular channels, square channels, or any other configuration suitable for the purposes described herein. Adjustment portion 644 is generally rectangular, with second channel 654 disposed in surface 657 and extending inward into adjustment portion 644 relative to surface 657.

Adjustment portion 644 further includes a thumbwheel 676, as shown in FIG. 20. Thumbwheel 676 is disposed within a cavity defined by adjustment portion 644. Thumbwheel 676 is disposed on a first pivot axis 684 such that thumbwheel 676 rotates about first pivot axis 684. An upper radius of thumbwheel 676 extends through a longitudinal slot defined by housing 636 for user manipulation. A lower radius of thumbwheel 676 extends through a longitudinal slot defined by adjustment portion 644 and into second channel 654, as shown in FIG. 21. Adjustment portion 644 is configured such that thumb wheel 676 abuts an outer surface of shaft 902 (FIG. 20) when adjustment mechanism 606 is in a first (closed) configuration, as described in greater detail below.

Adjustment portion 644 is generally constructed to provide selective retraction/advancement of a shaft 902 of delivery system 900 relative to introducer 904. Adjustment portion 644 can have a variety of constructions and/or devices capable of providing the desired user interface and the current embodiment shown in FIGS. 20-22 is not meant to limit the design, but rather provide an example of one possible embodiment.

Adjustment mechanism 606 includes the first configuration, shown in FIG. 20, wherein adjustment portion 644 is pivoted towards base 642 such that an outer radius of thumbwheel 676 contacts the outer surface of shaft 902. Adjustment mechanism 606 further includes a second configuration, shown in FIG. 21, wherein adjustment portion 644 is pivoted away from base 642 along hinge 665 such that the outer radius of thumbwheel 676 does not contact the outer surface of shaft 902 such that adjustment mechanism 606 may be disposed over or removed from shaft 902. Stated another way, adjustment portion 644 of adjustment mechanism 606 is configured to pivot away from base 642 to accept or remove shaft 902 therein (FIG. 21) and to pivot over base 642 to retain and manipulate shaft 902 (FIG. 20) therein.

With the above understanding of components of adjustment mechanism 606 in mind, operation and interaction of components of the present embodiment may be explained. FIG. 20 illustrates adjustment mechanism 606 in the first configuration such that, as thumbwheel 676 is rotated in a first direction D1, the outer radius of thumbwheel 676 engages shaft 902 such that thumbwheel 676 frictionally moves shaft 902 in a second direction D2. Movement of shaft 902 in second direction D2 is relative to introducer support 102 and adjustment mechanism 606 of the stability and adjustment mechanism. In the embodiment shown, directions D1 and D2 are opposite in the sense that pushing the exposed portion of thumbwheel 676 towards the distal end results in the shaft moving proximally. However, this is not meant to limit the design and gearing or other devices may be included such that pushing the exposed portion of thumbwheel 676 towards the distal end results in shaft 902 moving distally.

While only some embodiments according to the present invention have been described herein, it should be understood that they have been presented by way of illustration and example only, and not limitation. Various changes in form and detail can be made therein without departing from the scope of the invention.

The invention is defined by the following claims.

## Claims

1. A stabilization and advancement system (100) for direct transcatheter aortic valve implantation comprising:
an introducer support (108) configured to be coupled to an introducer (904) of a delivery system (900) and configured to be coupled to a first stable object (S1) in a procedure room;
a cradle mechanism (104) configured to be coupled to a handle (906) of the delivery system (900) and configured to be coupled to a second stable object (S2) in the procedure room;
an adjustment mechanism (106, 506, 606) configured to be coupled to the delivery system (900), wherein operation of the adjustment mechanism (106, 506, 606) moves the delivery system (900) distal of the handle (906) in a distal or proximal direction;
further comprising a flexible position clamp (109), wherein the introducer support (108) is configured to be coupled to the flexible position clamp (109) and the flexible position clamp (109) is configured to be coupled to the first stable object (S1).

2. The stabilization and advancement system of claim 1, wherein the flexible position clamp (109) comprises a plurality of arms (144), wherein adjacent arms (144) of the plurality of arms (144) are coupled to each other at flexible hinges (146).

3. The stabilization and advancement system of claim 2, wherein the flexible hinges (146) are lockable.

4. The stabilization and advancement system of claim 1, wherein the introducer support (108) comprises a lock mechanism (127) configured to couple the introducer support (108) to the adjustment mechanism (106, 506, 606).

5. The stabilization and advancement system of claim 1, wherein the cradle mechanism (104) includes:
a cradle (158) for attachment to the handle (906) of the delivery system (900);
a flexible arm (160) coupled to the cradle (158); and
a clamp (162) coupled to the flexible arm (160) at an end of the flexible arm (160) opposite the cradle (158), wherein the clamp (162) is configured to be attached to the second stable object (S2).

6. The stabilization and advancement system of claim 1, wherein the second stable object (S2) is the same as the first stable object (S1).

7. The stabilization and advancement system of claim 6, wherein the first stable object (S1) and the second stable object (S2) are a procedure table of the procedure room.

8. The stabilization and advancement system of claim 6, wherein the first stable object (S1) and the second stable object (S2) are both a procedure table of the procedure room.

9. The stabilization and advancement system of claim 1, wherein the adjustment mechanism (106, 506, 606) is configured to be coupled to the delivery system (900) adjacent the introducer (904).

10. The stabilization and advancement system of claim 1, wherein the adjustment mechanism (106, 506, 606) includes:
a housing (236, 536, 636);
a surface on an interior of the housing (236, 536, 636) configured to abut a shaft (902) of the delivery system (900) when the adjustment mechanism (106, 506, 606) is coupled to the delivery system (900);
a wheel (264, 564) configured to abut the shaft (902) of the delivery system (900) opposite the surface; and
a thumbwheel (276, 576, 676) extending at least partially outside of the housing (236, 536, 636), wherein the thumbwheel (276, 576, 676) is coupled to the wheel (264, 564) such that when the thumbwheel (276, 576, 676) in moved in a first direction, the wheel (264, 564) and the surface frictionally move the shaft (902) in the first direction.

11. The stabilization and advancement system of claim 10, wherein the adjustment mechanism (106, 506, 606) further includes a gear coupled between the thumbwheel (276, 576, 676) and the wheel (264, 564).

12. The stabilization and advancement system of claim 10, wherein the adjustment mechanism (106, 506, 606) further includes a quick release mechanism such that the adjustment mechanism (106, 506, 606) can be removed from the delivery system (900).

13. The stabilization and advancement system of claim 10, wherein the adjustment mechanism (106, 506, 606) further includes a tube (238, 538) extending distally from the housing (236, 536, 636), wherein the tube (238, 538) is configured to be disposed within and coupled to the introducer support (108).

## Patentansprüche

1. Stabilisierungs- und Vorschubsystem (100) für eine direkte Transkatheter-Aortenklappenimplantation, das Folgendes umfasst:
eine Einführhilfe (108), die konfiguriert ist, um mit einem Einführer (904) eines Abgabesystems (900) gekoppelt zu werden, und konfiguriert ist, um mit einem ersten stabilen Objekt (S1) in einem Operationssaal gekoppelt zu werden;
einen Halterungsmechanismus (104), der konfiguriert ist, um mit einem Griff (906) des Abgabesystems (900) gekoppelt zu werden, und konfiguriert ist, um mit einem zweiten stabilen Objekt (S2) in dem Operationssaal gekoppelt zu werden;
einen Einstellmechanismus (106, 506, 606), der konfiguriert ist, um mit dem Abgabesystem (900) gekoppelt zu werden, wobei ein Betrieb des Einstellmechanismus (106, 506, 606) das Abgabesystem (900) distal von dem Griff (906) in eine distale oder eine proximale Richtung bewegt;
das ferner eine flexible Positionsklemme (109) umfasst, wobei die Einführhilfe (108) konfiguriert ist, um mit der flexiblen Positionsklemme (109) gekoppelt zu werden, und die flexible Positionsklemme (109) konfiguriert ist, um mit dem ersten stabilen Objekt (S1) gekoppelt zu werden.

2. Stabilisierungs- und Vorschubsystem nach Anspruch 1, wobei die flexible Positionsklemme (109) mehrere Arme (144) umfasst, wobei benachbarte Arme (144) der mehreren Arme (144) an flexiblen Gelenken (146) miteinander gekoppelt sind.

3. Stabilisierungs- und Vorschubsystem nach Anspruch 2, wobei die flexiblen Gelenke (146) verriegelbar sind.

4. Stabilisierungs- und Vorschubsystem nach Anspruch 1, wobei die Einführhilfe (108) einen Verriegelungsmechanismus (127) umfasst, der konfiguriert ist, um die Einführhilfe (108) mit dem Einstellmechanismus (106, 506, 606) zu koppeln.

5. Stabilisierungs- und Vorschubsystem nach Anspruch 1, wobei der Halterungsmechanismus (104) Folgendes beinhaltet:
eine Halterung (158) für eine Befestigung an dem Griff (906) des Abgabesystems (900);
einen flexiblen Arm (160), der mit der Halterung (158) gekoppelt ist; und
eine Klemme (162), die an einem Ende des flexiblen Arms (160) gegenüber der Halterung (158) mit dem flexiblen Arm (160) gekoppelt ist, wobei die Klemme (162) konfiguriert ist, um an dem zweiten stabilen Objekt (S2) befestigt zu werden.

6. Stabilisierungs- und Vorschubsystem nach Anspruch 1, wobei das zweite stabile Objekt (S2) das gleiche wie das erste stabile Objekt (S1) ist.

7. Stabilisierungs- und Vorschubsystem nach Anspruch 6, wobei das erste stabile Objekt (S1) und das zweite stabile Objekt (S2) ein Operationstisch des Operationssaals sind.

8. Stabilisierungs- und Vorschubsystem nach Anspruch 6, wobei das erste stabile Objekt (S1) und das zweite stabile Objekt (S2) beide ein Operationstisch des Operationssaals sind.

9. Stabilisierungs- und Vorschubsystem nach Anspruch 1, wobei der Einstellmechanismus (106, 506, 606) konfiguriert ist, um mit dem Abgabesystem (900) angrenzend an den Einführer (904) gekoppelt zu werden.

10. Stabilisierungs- und Vorschubsystem nach Anspruch 1, wobei der Einstellmechanismus (106, 506, 606) Folgendes beinhaltet:
ein Gehäuse (236, 536, 636);
eine Oberfläche auf einem Inneren des Gehäuses (236, 536, 636), die konfiguriert ist, um an einem Schaft (902) des Abgabesystems (900) anzugrenzen, wenn der Einstellmechanismus (106, 506, 606) mit dem Abgabesystem (900) gekoppelt ist;
ein Rad (264, 564), das konfiguriert ist, um an dem Schaft (902) des Abgabesystems (900) gegenüber der Oberfläche anzugrenzen; und
ein Daumenrad (276, 576, 676), das sich wenigstens teilweise außerhalb des Gehäuses (236, 536, 636) erstreckt, wobei das Daumenrad (276, 576, 676) mit dem Rad (264, 564) derart gekoppelt ist, dass, wenn das Daumenrad (276, 576, 676) in eine erste Richtung bewegt wird, das Rad (264, 564) und die Oberfläche den Schaft (902) reibschlüssig in die erste Richtung bewegen.

11. Stabilisierungs- und Vorschubsystem nach Anspruch 10, wobei der Einstellmechanismus (106, 506, 606) ferner ein Zahnrad beinhaltet, das zwischen dem Daumenrad (276, 576, 676) und dem Rad (264, 564) gekoppelt ist.

12. Stabilisierungs- und Vorschubsystem nach Anspruch 10, wobei der Einstellmechanismus (106, 506, 606) ferner einen Schnelllösemechanismus derart beinhaltet, dass der Einstellmechanismus (106, 506, 606) aus dem Abgabesystem (900) entfernt werden kann.

13. Stabilisierungs- und Vorschubsystem nach Anspruch 10, wobei der Einstellmechanismus (106, 506, 606) ferner ein Rohr (238, 538) beinhaltet, das sich distal von dem Gehäuse (236, 536, 636) erstreckt, wobei das Rohr (238, 538) konfiguriert ist, um innerhalb der Einführhilfe (108) angeordnet und mit dieser gekoppelt zu sein.

## Revendications

1. Système de stabilisation et d'avancement (100) pour implantation de valvule aortique par voie transcathéter comprenant :
un support d'introducteur (108) conçu pour être accouplé à un introducteur (904) d'un système d'administration (900) et conçu pour être accouplé à un premier objet stable (S1) dans une salle d'opération ;
un mécanisme de berceau (104) conçu pour être accouplé à une poignée (906) du système d'administration (900) et conçu pour être accouplé à un second objet stable (S2) dans la salle d'opération ;
un mécanisme de réglage (106, 506, 606) conçu pour être accouplé au système d'administration (900), le fonctionnement du mécanisme de réglage (106, 506, 606) déplaçant le système d'administration (900) de manière distale de la poignée (906) en une direction distale ou proximale ;
comprenant en outre une pince de position flexible (109), le support d'introducteur (108) étant conçu pour être accouplé à la pince de position flexible (109) et la pince de position flexible (109) étant conçue pour être accouplée au premier objet stable (S1).

2. Système de stabilisation et d'avancement selon la revendication 1, dans lequel la pince de position flexible (109) comprend une pluralité de bras (144), les bras adjacents (144) de la pluralité de bras (144) étant accouplés les uns aux autres au niveau de charnières flexibles (146).

3. Système de stabilisation et d'avancement selon la revendication 2, dans lequel les charnières flexibles (146) sont verrouillables.

4. Système de stabilisation et d'avancement selon la revendication 1, dans lequel le support d'introducteur (108) comprend un mécanisme de verrouillage (127) conçu pour accoupler le support d'introduction (108) au mécanisme de réglage (106, 506, 606).

5. Système de stabilisation et d'avancement selon la revendication 1, dans lequel le mécanisme de berceau (104) comporte :
un berceau (158) pour la fixation à la poignée (906) du système d'administration (900) ;
un bras flexible (160) accouplé au berceau (158) ; et
une pince (162) accouplée au bras flexible (160) à une extrémité du bras flexible (160) opposée au berceau (158), la pince (162) étant conçue pour être fixée au second objet stable (S2).

6. Système de stabilisation et d'avancement selon la revendication 1, dans lequel le second objet stable (S2) est le même que le premier objet stable (S1).

7. Système de stabilisation et d'avancement selon la revendication 6, dans lequel le premier objet stable (S1) et le second objet stable (S2) sont une table d'opération de la salle d'opération.

8. Système de stabilisation et d'avancement selon la revendication 6, dans lequel le premier objet stable (S1) et le second objet stable (S2) sont tous deux une table d'opération de la salle d'opération.

9. Système de stabilisation et d'avancement selon la revendication 1, dans lequel le mécanisme de réglage (106, 506, 606) est conçu pour être accouplé au système d'administration (900) adjacent à l'introducteur (904).

10. Système de stabilisation et d'avancement selon la revendication 1, dans lequel le mécanisme de réglage (106, 506, 606) comporte :
un boîtier (236, 536, 636) ;
une surface à l'intérieur du boîtier (236, 536, 636) conçue pour venir en butée contre un arbre (902) du système d'administration (900) lorsque le mécanisme de réglage (106, 506, 606) est accouplé au système d'administration (900) ;
une roue (264, 564) conçue pour venir en butée contre l'arbre (902) du système d'administration (900) à l'opposé de la surface ; et
une molette (276, 576, 676) s'étendant au moins partiellement à l'extérieur du boîtier (236, 536, 636), la molette (276, 576, 676) étant accouplée à la roue (264, 564) de sorte que lorsque la molette (276, 576, 676) se déplace dans une première direction, la roue (264, 564) et la surface déplaçant par friction l'arbre (902) dans la première direction.

11. Système de stabilisation et d'avancement selon la revendication 10, dans lequel le mécanisme de réglage (106, 506, 606) comporte en outre un engrenage accouplé entre la molette (276, 576, 676) et la roue (264, 564).

12. Système de stabilisation et d'avancement selon la revendication 10, dans lequel le mécanisme de réglage (106, 506, 606) comporte en outre un mécanisme de libération rapide de sorte que le mécanisme de réglage (106, 506, 606) peut être retiré du système d'administration (900).

13. Système de stabilisation et d'avancement selon la revendication 10, dans lequel le mécanisme de réglage (106, 506, 606) comporte en outre un tube (238, 538) s'étendant de manière distale depuis le boîtier (236, 536, 636), dans lequel le tube (238, 538) est conçu pour être disposé à l'intérieur et accouplé au support d'introducteur (108).
